(19) Europäisches Patentamt
European Patent Office
Office européen des brevets

(11) Numéro de publication : **0 083 274**
**B1**

(12) **FASCICULE DE BREVET EUROPÉEN**

(45) Date de publication du fascicule du brevet : **24.04.85**

(51) Int. Cl.⁴ : **C 07 C 21/22**, C 07 C 17/00

(21) Numéro de dépôt : **82402329.5**

(22) Date de dépôt : **17.12.82**

(54) **Procédé de préparation d'hydrocarbures acétyléniques vrais à chaîne perfluorée.**

(30) Priorité : **29.12.81 FR 8124364**

(43) Date de publication de la demande : **06.07.83 Bulletin 83/27**

(45) Mention de la délivrance du brevet : **24.04.85 Bulletin 85/17**

(84) Etats contractants désignés : **BE CH DE FR GB IT LI NL SE**

(56) Documents cités : **FR-A- 2 307 784**

(73) Titulaire : **ATOCHEM**
**12/16, allée des Vosges**
**F-92400 Courbevoie (FR)**

(72) Inventeur : **Calas, Patrick**
**24, rue Durand**
**F-34000 Montpellier (FR)**
Inventeur : **Moreau, Patrice**
**30, rue des Oliviers**
**F-34980 Saint-Gely du Fesc (FR)**
Inventeur : **Commeyras, Auguste**
**6 Impasse des Ecoles**
**F-34960 Claplers (FR)**

(74) Mandataire : **Rochet, Michel et al**
**ATOCHEM Département Propriété Industrielle Cedex 22**
**F-92091 Paris la Défense (FR)**

**EP 0 083 274 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un procédé de préparation d'hydrocarbures acétyléniques à chaîne perfluorée de formule générale $R_F$—C≡CH que l'on désigne sous le nom d'acétyléniques vrais, et dans laquelle $R_F$ désigne un radical perfluoroalkyle $C_nF_{2n+1}$ — à chaîne droite ou ramifiée. Ces composés sont des intermédiaires utilisés pour introduire des chaînes perfluoroalkyle dans les molécules organiques.

Les produits dans lesquels n est 1, 2 ou 3 (c'est-à-dire $R_F$ est $CF_3$, $C_2F_5$ ou $C_3F_7$) sont connus par les articles de A. L. Henne et M. Nager, J. Amer. Chem. Soc. 1951, 73 1042 et de R. N. Haszeldine et K. Leedham J. Chem. Soc. 1952, 3483. Les composés à chaîne perfluoroalkyle plus longue ont été préparés plus récemment par des réactions successives de bromation, déhydrobromation et débromation, par exemple par M. Le Blanc et coll. J. Fluorine Chem. 1976, 7, 525, ou par une réaction initiée par des couples à base de cuivre entre des iodures de perfluoroalkyle $R_FI$ et des substrats insaturés, décrite dans J. Org. Chem. 1975, 40, 810 mais ces procédés nécessitent des étapes nombreuses qui conduisent à des rendements faibles.

La demanderesse a mis au point un procédé qui permet de préparer les hydrocarbures acétyléniques vrais à chaîne perfluorée avec des rendements excellents à partir de produits faciles à obtenir. Ce procédé consiste à faire réagir sur un iodure de perfluoroalkyle un alcool acétylénique tertiaire puis après déhydroiodation de l'alcool acétylénique obtenu, la distillation sur soude solide de l'alcool acétylénique perfluoré conduit à un mélange de l'hydrocarbure acétylénique vrai perfluoré et d'une cétone. Les diverses réactions sont les suivantes :

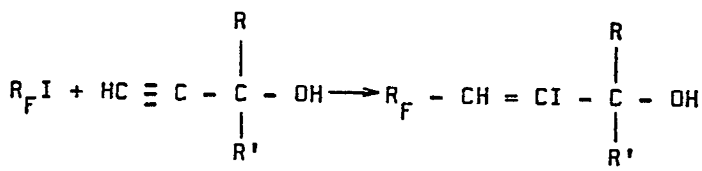

(I)

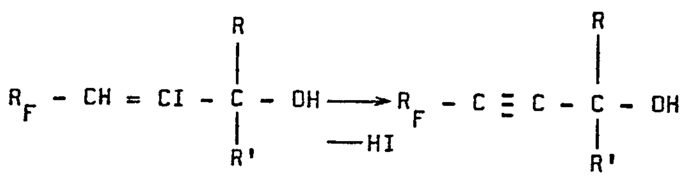


(II)

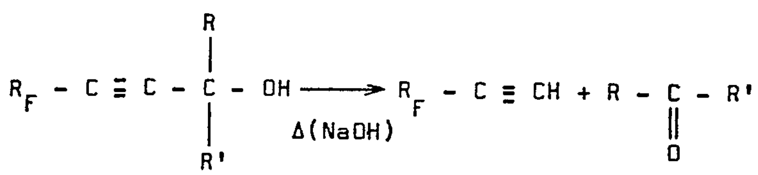


(III)

R et R' représentent des radicaux alkyles identiques ou différents.

Le rapport molaire $R_FI$/alcool acétylénique peut varier de 3/1 à 1/5. Pour la réaction II, le rapport molaire iodhydrine/KOH varie de 1/1 à 1/5. Pour ces deux réactions, la température peut aller de 20 à 100 °C. La distillation au cours de laquelle se produit la réaction III a lieu avec élévation de température.

La préparation de la iodhydrine peut se faire par électrocatalyse suivant la demande de brevet FR 80.15121 ou par catalyse à l'aide de couples $Hg^{++}/Hg^{+}$ ou $Mn^{3+}/Mn^{++}$ selon la demande de brevet FR 80.15122. Dans le cas considéré où R et R' sont des radicaux alkyle, la iodhydrine obtenue est exclusivement sous la forme trans, la seule qui puisse conduire au produit (II) ce qui peut se faire soit directement dans la cellule d'électrolyse en laissant le pH de la cathode augmenter, soit de préférence en la traitant par de la potasse en milieu alcoolique, avec un rendement de l'ordre de 95 %. L'acétylénique perfluoré est obtenu par distillation du produit II sur de la soude solide, la cétone sous-produite étant séparée au cours de la distillation.

Les exemples suivants illustrent l'invention sans toutefois la limiter.

Exemple 1

On utilise une cellule en verre à deux compartiments dont le schéma se trouve sur la figure en annexe.

Chaque compartiment est un cylindre d'axe horizontal de 5 cm de diamètre et 5 cm de hauteur et

d'un volume de 100 ml. Les deux compartiments sont séparés par un verre fritté (3) de 5 cm de diamètre et de porosité 3.

Le compartiment cathodique (7) reçoit une cathode (2) constituée par 60 cm de mèche de fibre de carbone présentant 10 000 fibres de 3 μ par mèche, telle par exemple la Rigilor AGTF 10 000, marque déposée de la Société Carbone-Lorraine, connectée au circuit intérieur par des fils de cuivre non immergés. Ce compartiment reçoit en outre un barreau magnétique d'agitation (4) placé dans un plan vertical et séparé des fibres de carbone par une grille en nylon ayant des mailles de 2 mm. Ce barreau est entraîné par un agitateur (5).

Le compartiment anodique (6) comporte une anode de carbone (1) constituée par un barreau vertical de 6 mm de diamètre.

Les deux électrodes sont reliées à un potentiostat monté en intensiostat.

On introduit dans le compartiment cathodique 100 g de $C_6F_{13}I$ mélangés à 18,8 g de H—C≡C—C $(CH_3)_2OH$ (rapport molaire 1 : 1) et 30 ml d'eau saturée en KCl. Le compartiment anodique est garni avec 100 ml d'eau saturée de KCl.

L'électrolyse est conduite sous un ampérage imposé de 0,7 ampère. La chute ohmique dans la cellule est de l'ordre de 11 volts.

Au bout de deux heures, l'électrolyse est stoppée, le catholyte déchargé et lavé à trois reprises avec 200 ml d'eau. La phase organique lourde est séparée par décantation. Son analyse révèle la présence de 80 g de l'iodhydrine trans $C_6F_{13}$—CH=CI—$C(CH_3)_2OH$, de 30 g de $C_6F_{13}I$ non transformé et de traces de l'alcool acétylénique $C_6F_{13}$—C≡C—$C(CH_3)_2$—OH. Le rendement en iodhydrine par rapport à l'iodure de perfluoroalkyle de départ transformé est de 98 %. Elle est purifiée par distillation sous vide.

Le rendement faradique de l'électrolyse est de 0,3 Faraday par mole de $C_6F_{13}I$ converti.

A 50 g de l'iodhydrine obtenue dissous dans 200 ml de méthanol sont ajoutés 7,6 g d'hydroxyde de potassium KOH, ce qui correspond à un rapport molaire KOH/iodhydrine de 1,5. Le mélange est laissé deux heures à la température ambiante puis lavé trois fois avec 500 ml d'eau. Une couche incolore de 37,9 g se sépare, constituée de l'acétylénique $C_6F_{13}$—C≡C—$(CH_3)_2OH$ ce qui représente un rendement quantitatif.

Dans un claisen de 25 ml sont introduits 10 g de cet acétylénique et 3 pastilles de soude NaOH. On distille lentement et recueille un produit qui distille à partir de 95 °C, le chauffage étant maintenu jusqu'au passage de vapeurs à 180 °C.

Le ballon de réception contient un mélange équimolaire d'acétone et de 5,6 g de l'acétylénique $C_6F_{13}$—C≡CH qui sont séparés par distillation, soit un rendement de 65 % pour la réaction de coupure.

## Exemples 2 à 4

On opère comme dans l'exemple 1, mais avec des produits ayant des radicaux variés. Le tableau ci-dessous indique les résultats obtenus.

| Produits de départ | | | Acétylénique perfluoré | |
|---|---|---|---|---|
| $R_F$ | R | R' | Point d'ébullition (°C) | Rendement de la distillation |
| $C_4F_9$ | $CH_3$ | $CH_3$ | 42 | 80 |
| $C_4F_9$ | $CH_3$ | $C_2H_5$ | 42 | 65 |
| $C_6F_{13}$ | $CH_3$ | $C_2H_5$ | 94 - 96 | 70 |

## Exemple 5

Dans un réacteur de 250 ml muni d'un agitateur magnétique, on introduit 0,1 mole (8,4 g) d'alcool H—C≡C—$C(CH_3)_2OH$, 5 ml de DMF, $1,5 \cdot 10^{-3}$ mole (0,98 g) de $Hg_2I_2$, $6\ 10^{-3}$ mole (2,72 g) de $HgI_2$ et 0,02 mole (8,92 g) de $C_6F_{13}I$. Le mélange est chauffé à 35-40 °C pendant 72 heures. Au bout de ce temps, la totalité du $C_6F_{13}I$ est transformée. Le rendement en iodhydrine, vérifié par CPV et RMN ($H^1$ et $F^{19}$) est quantitatif. Le produit est séparé en versant le mélange réactionnel dans 250 ml d'eau. Une couche dense se forme, qui est séparée et extraite avec du $CCl_4$. Après séchage et évaporation du $CCl_4$, on obtient 9,2 g de iodhydrine (rendement 86,8 %) qui peut être engagée dans la suite des réactions décrites dans l'exemple 1, pour donner l'acétylénique vrai correspondant.

**Revendications**

1. Procédé de préparation des hydrocarbures acétyléniques vrais à chaîne perfluorée caractérisé par une déhydroiodation par la potasse en milieu alcoolique de la iodhydrine.

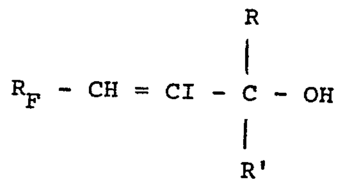

où $R_F$ est une chaîne perfluorée $C_nF_{2n+1}$ droite ou ramifiée et R et R′ sont des radicaux alkyles identiques ou différents, suivie de la distillation en présence de soude solide de l'alcool acétylénique obtenu.

2. Procédé selon la revendication 1 dans lequel la iodhydrine utilisée est préparée par électrocatalyse.

3. Procédé selon la revendication 1 dans lequel la iodhydrine utilisée est préparée par catalyse à l'aide de couples mercureux/mercurique ou manganeux/manganique.

**Claims**

1. Process for the preparation of true acetylenic hydrocarbons having a perfluorinated chain, characterised by a dehydroiodination of the iodohydrin

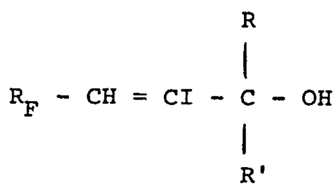

where $R_F$ is a straight or branched perfluorinated chain $C_nF_{2n+1}$ and R and R′ are identical or different alkyl radicals, by means of potassium hydroxide in an alcoholic medium, followed by distillation, in the presence of solid sodium hydroxide, of the acetylenic alcohol obtained.

2. Process according to claim 1 in which the iodohydrin used is prepared by electrocatalysis.

3. Process according to claim 1 in which the iodohydrin used is prepared by catalysis by means of the mercurous/mercuric or manganous/manganic couple.

**Patentansprüche**

1. Verfahren zur Herstellung von echten acetylenischen Kohlenwasserstoffen mit perfluorierter Kette, gekennzeichnet durch eine Jodwasserstoffabspaltung in alkoholischer Lösung mittels Kaliumhydroxid aus dem Jodhydrin

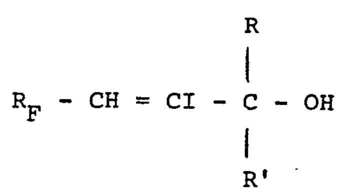

worin $R_F$ eine gerade oder verzweigte perfluorierte Kette $C_nF_{2n+1}$ und R und R′ gleiche oder verschiedene Alkylradikale sind, und durch nachfolgende Destillation des gebildeten acetylenischen Alkohols in Gegenwart von festem Natriumhydroxid.

2. Verfahren nach Anspruch 1, in dem das eingesetzte Jodhydrin durch Elektrokatalyse hergestellt wird.

3. Verfahren nach Anspruch 1, in dem das eingesetzte Jodhydrin durch Katalyse mittels der Paare $Hg^{++}/Hg^{+}$ oder $Mn^{3+}/Mn^{2+}$ hergestellt wird.

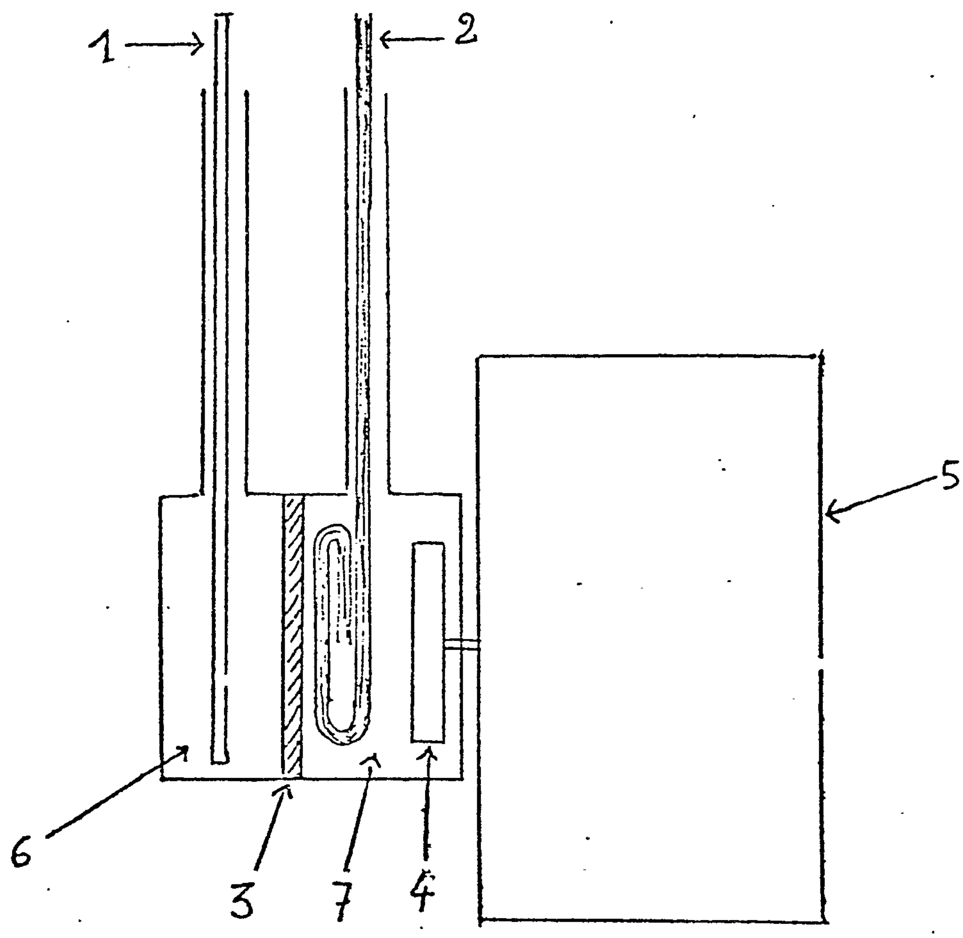
1
2
5
6
3
7
4